(19) Europäisches Patentamt
European Patent Office
Office européen des brevets

(11) **EP 3 720 461 B1**

(12) **EUROPEAN PATENT SPECIFICATION**

(45) Date of publication and mention
of the grant of the patent:
**08.02.2023 Bulletin 2023/06**

(21) Application number: **18836801.3**

(22) Date of filing: **05.12.2018**

(51) International Patent Classification (IPC):
**A61K 36/185** (2006.01)    **A61K 36/24** (2006.01)
**A61P 17/02** (2000.01)    **A61P 29/00** (2000.01)

(52) Cooperative Patent Classification (CPC):
(C-Sets available)
**A61K 36/185; A61K 36/24; A61P 17/02;**
**A61P 29/00;** A61K 2236/331    (Cont.)

(86) International application number:
**PCT/ZA2018/050064**

(87) International publication number:
**WO 2019/113614 (13.06.2019 Gazette 2019/24)**

(54) **A PLANT-DERIVED MEDICINAL COMPOSITION**

MEDIZINISCHE ZUSAMMENSETZUNG AUS PFLANZEN

COMPOSITION MÉDICINALE DÉRIVÉE D'UNE PLANTE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.12.2017 ZA 201708330**

(43) Date of publication of application:
**14.10.2020 Bulletin 2020/42**

(73) Proprietors:
• **TSHWANE UNIVERSITY OF TECHNOLOGY**
  **0183 Pretoria (ZA)**
• **Global Health Biotech (PTY) Ltd**
  **0087 Pretoria (ZA)**

(72) Inventors:
• **MAEPA, Makwese**
  **Sunnyside**
  **0002 Pretoria (ZA)**
• **MPILU, Johanna**
  **0920 Louis Trichardt (ZA)**
• **RAZWINANI, Mapula**
  **0083 Pretoria (ZA)**
• **MOTAUNG, Keo**
  **0008 Pretoria (ZA)**

(74) Representative: **Ellis, Michael James**
**Ellis IP Ltd**
**53/4 George Street**
**Edinburgh EH2 2HT (GB)**

(56) References cited:
• **STEENKAMP V ET AL: "Studies on antibacterial, antioxidant and fibroblast growth stimulation of wound healing remedies from South Africa", JOURNAL OF ETHNOPHARMACO, ELSEVIER IRELAND LTD, IE, vol. 95, no. 2-3, 1 December 2004 (2004-12-01), pages 353-357, XP004990588, ISSN: 0378-8741, DOI: 10.1016/J.JEP.2004.08.020**
• **RAGUPATHY SUBRAMANYAM ET AL: "Valorizing the 'Irulas' traditional knowledge of medicinal plants in the Kodiakkarai Reserve Forest, India", JOURNAL OF ETHNOBIOLOGY AND ETHNOMEDICINE, BIOMED CENTRAL, LONDON, GB, vol. 5, no. 1, 14 April 2009 (2009-04-14), page 10, XP021051970, ISSN: 1746-4269, DOI: 10.1186/1746-4269-5-10**
• **KN REDDY 1* ET AL: "Medicinal plants used by ethnic people of Medak district, Andhra Pradesh", INDIAN JOURNAL OF TRADITIONAL KNOWLEDGE, RESOURCES, NEW DELHI, NEW DELHI - INDIA, vol. 9, no. 1, 1 January 2010 (2010-01-01) , pages 184-190, XP018027603, ISSN: 0972-5938**

- **MOHAGHEGHZADEH A ET AL: "Medicinal smokes", JOURNAL OF ETHNOPHARMACOLOGY, ELSEVIER IRELAND LTD, IE, vol. 108, no. 2, 24 November 2006 (2006-11-24), pages 161-184, XP027939753, ISSN: 0378-8741 [retrieved on 2006-11-24]**
- **H.C. ONG ET AL: "Malay ethno-medico botany in Machang, Kelantan, Malaysia", FITOTERAPIA., vol. 70, no. 5, 1 October 1999 (1999-10-01), pages 502-513, XP055243682, IT ISSN: 0367-326X, DOI: 10.1016/S0367-326X(99)00077-5**

(52) Cooperative Patent Classification (CPC): (Cont.)

C-Sets
**A61K 36/185, A61K 2300/00;
A61K 36/24, A61K 2300/00**

**Description**

**Field of the Invention**

This invention relates to a plant-derived medicinal composition which is believed to have anti-inflammatory properties as well as the ability to promote collagen type 11 formation in joints.

**Background to the Invention**

Many pharmaceuticals for treating inflammation in the human or animal body are currently available. Unbalanced and prolonged inflammation leads to progressive tissue damage and has been implicated in the development of chronic diseases. It is therefore important to treat inflammation.

This is usually achieved by the use of nonsteroidal anti-inflammatory drugs that reduce pain, decrease fever, and, in higher doses, decrease inflammation. Side effects however include an increased risk of stomach ulcers, thrombosis, and heart attacks.

It is therefore desirable to develop an alternative treatment for inflammation that has fewer, less severe, or no side effects when compared to nonsteroidal anti-inflammatory drugs.

**Summary of the Invention**

According to the invention, there is provided a medicinal composition having anti-inflammatory properties including an extract derived from the root and bark of *Capparis Sepiaria* and *Tabernaemontana Elegans.*

If the composition is intended for topical application, it may further include:

- a carbomer;

- water;

- a preservative;

- Ethanol; and

- Propylene glycol.

The preservative may be DMDM Hydantoin.

The above constituents may be combined and then mixed with a suitable carrier for use.

The carrier may be selected from the group including: a gel, a spray, a paste, and a cream as are commonly used in cosmetic applications.

In order to prepare the extract used in the medicinal composition, root and bark of *Capparis Sepi*aria and *Tabernaemontana Elegans* may be harvested, washed with water and allowed to dry at room temperature for about two weeks.

Typically, the two plants are used in equal measures of which 50 % by weight will be root and 50 % by weight bark.

Thereafter, the bark and root may be mixed and ground in, for example, a Wiley mill grinder.

The resulting powder may be mixed with 50 ml distilled water in a glass container.

The solution may be placed in a shaker for about 24 hours after which it may filtered using, for example, man fitter filter paper.

The filtrate may be frozen at a temperature of about - 70 °C after which it may be placed in a lyophiliser for four days, following which it may be ready for use.

When used as a topical application, the medicinal composition may include the following constituents, in mass percent:

Active Extract: 0.252 %;

Carbomer: 0.855%;

Water: 69.503%;

DMDM Hydantoin: 0.26 7%;

Ethanol 95 M5: 21.386 %; and

Propylene glycol: 7.485%.

The above mixture may then be combined with a carrier such as, for example, a gel.

The mass percentage of the above mixture may in this case be 99.14457 and that of the gel 0.85542.

This topical application may be used to relieve/assist one or more of: muscle aches, joint aches, localised pain management, and wound healing.

It is further believed that the extract stimulates the expression of collagen type II, thereby aiding the regeneration of damaged cartilage in joints.

**Investigative Experiments**

Background:

Macrophages are a major component of the mononuclear phagocyte system that consists of closely related cells of bone marrow origin, including blood monocytes, and tissue macrophages. From the blood, monocytes migrate into various tissues and transform into macrophages.

In inflammation, macrophages have three major functions, namely: antigen presentation, phagocytosis, and immunomodulation through production of various cytokines and growth factors. Macrophages thus play a critical role in the initiation, maintenance, and resolution of inflammation. They are activated and deactivated in the inflammatory process.

Activation signals include cytokines (interferon γ, granulocyte-monocyte colony stimulating factor, and

tumour necrosis factor $\alpha$), bacterial lipopolysaccharide, extracellular matrix proteins, and other chemical mediators. Inhibition of inflammation by removal or deactivation of mediators and inflammatory effector cells permits the host to repair damages tissues.

Activated macrophages are deactivated by anti-inflammatory cytokines (interleukin 10 and transforming growth factor ß) (TGF- ß is the main deactivator) and cytokine antagonists that are mainly produced by macrophages. Macrophages participate in the autoregulatory loop in the inflammatory process.

Macrophages that are signalled to create an inflammatory response are M1 cells and the opposite are M2 cells, the difference between the two cells are in the metabolism of arginine (amino acid). M1 will metabolise to produce NO which is the effector chemical causing inflammation. M2 will metabolise to produce ornithine which will encourage repair of tissue.

Experiment 1: investigation of fatal toxicity levels based on a viability test of cells in culture

Murine monocyte/macrophage RAW264.7 cells were purchased from American Type Culture Collection (ATCC, USA) and sub cultured to confluence in Dulbecco's modified Eagle's medium (DMEM; Life Technologies, USA) containing 10% fetal bovine serum (FBS; Life Technologies, USA), penicillin (100 U/mL; Life Technologies, USA) and streptomycin (100 $\mu$g/mL; Life Technologies, USA) in a humidified 5% CO2 atmosphere at 37°C.

The tested extract (as described above) was reconstituted with water and 99% ethanol respectively at the following concentrations: 0, 6.25, 12.5, 25, 50, 100 and 200 $\mu$g/ml. This was then added to the cells being sub-cultured on growth medium.

The effects of the extract on the cells were expressed as a percentage of the blank controls.

Cell viability was determined by the 3-[4, 5-dimethylthiazol-2-yl]-2, 5-diphenyltetrazolium bromide (MTT; Sigma, USA) assay after 24, 48, and 72 hrs of treatment at varying levels of reconstituted extract concentrations in 96-well plates.

The relative amount of viable cells was determined by measuring the reduction of MTT dye in live cells to blue formazan crystals at optical density at 540 nm and expressed as the percentage of solvent control samples without exposure to the extract. The percentage of cell density for cells exposed to the extract was calculated by the following ratio:

$$\text{\% viability of cells} = ((A1\text{-}A2)/A0) \text{ X } 100$$

$$= \text{\% viable cells}$$

It was found that when the extract was reconstituted with water, concentrations above 12.5 $\mu$g/ml are toxic to cells when cultured for 72 hrs. No concentration showed toxicity when cultured for 24 hrs and 48hrs.

It was found that when the extract was reconstituted with ethanol, none of the investigated concentrations were toxic to cells when cultured for 24hrs, 48 hrs and 72 hrs.

Experiment 2: optimal cell proliferation at varying extract concentrations

The effect of the extract (as described above) on the proliferation and behaviour of RAW264.7 cells was evaluated by xCELLigence assay (RTCA DP Instrument, ACEA Biosciences, Inc.). In the xCELLigence system, cells are seeded in a special plate (E-plate 16 with micro-electrodes covering well bottoms). Briefly, the background of the E-plates 16 was determined in 100 $\mu$l medium and subsequently 200 $\mu$l of RAW264.7 cells was added (5 $\times$ 104 cells/ml).

Cells were incubated for 30 minutes at room temperature in the laminar floor and the E-plates were placed into the RTCA station in the incubator at 37°C in a humid atmosphere of 5% carbon dioxide. Cells were grown for 24 hours, with impedance measured every 30 minutes. After 24 hours, RAW264.7 cells were sub-cultured in DMEM containing 1% FBS and in the absence and presence of various concentrations of the extract when reconstituted with water and ethanol, respectively and monitored every 30 minutes for two, four and eight days.

Using the time- dependent cells response, impedance was represented by the cell index (CI) values and the cell viability rate was obtained from the CI curves. The CI value at each time point was defined as (Rt-Rb)/15 where Rt was defined as the cell-electrode impedance of the well with the cells at different time points, and Rb was defined as the background impedance of the well with the media alone. The normalised cell index was calculated by dividing the cell index value at a particular time point by the cell index value at the time of interest.

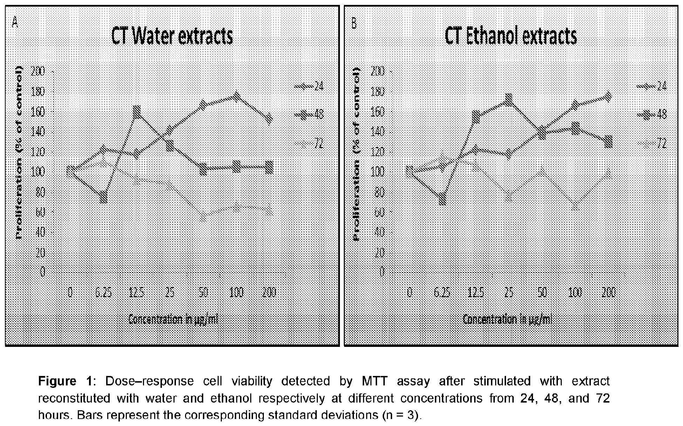

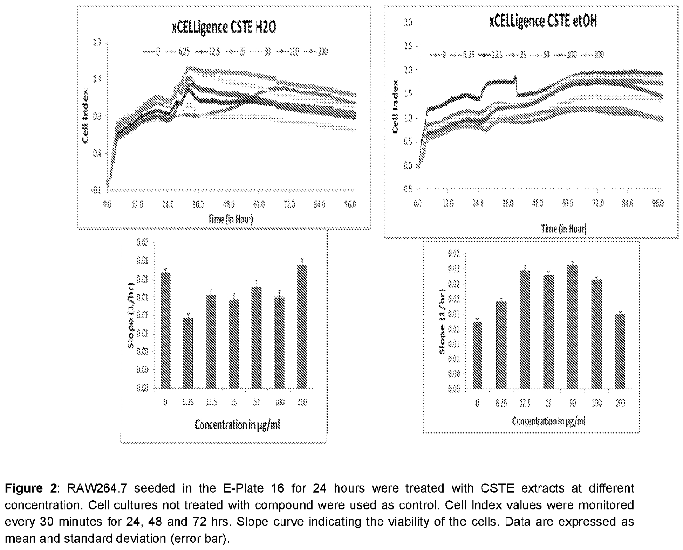

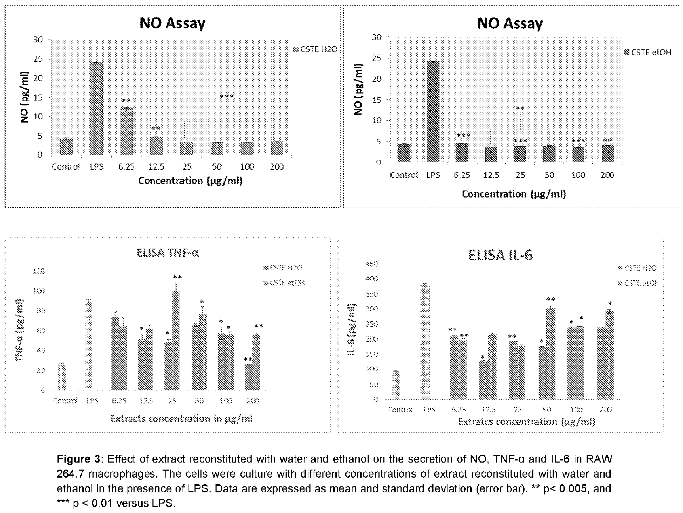

The xCELLigence system was used to monitor cell behaviour by allowing cell viability to be measured continuously and in real-time. The kinetic profile of cells exposed to extract reconstituted with water and ethanol respectively at different concentrations is shown in figures 2 and 3. The cells were monitored every 30 minutes for the indicated period of time. Kinetic profiles provided by the xCELLigence system indicated that the rate and dynamic of proliferation varied significantly between exposure types.

As expected, a subset of cells exposed to extract reconstituted with ethanol at 12.5 $\mu$g/ml concentrations showed mediated proliferation until the end of the experiment with a CI of 2.0 higher than that of the control cells. In contrast, extract reconstituted with water at a concentration of 6.25 $\mu$g/ml induced proliferation starting at 48 hrs (Fig.2) with CI equivalent to control cells.

Experiment 3: effect of the extract on anti-inflammatory responses of macrophage cells

3.1 Nitric Oxide is an effector chemical that induces inflammation. Cells were stimulated using LPS to respond in an inflammatory manner and thus produce NO. (NO) Inhibitory Assay: Macrophage cells RAW264.7 ($4 \times 105$ per well) were seeded. With 0.1 $\mu$g of lipopolysaccharide (LPS) per mL of medium, extract (as described above) reconstituted with water or ethanol respectively was added (at concentrations ranging from 0 to 200 $\mu$g/mL) and incubated for 24h. The nitrite accumulation in the supernatant was determined by Griess reagent (Sigma, St. Louis, MO, USA) [22, 23] read at a wavelength of 540 nm.

The extract reconstituted with ethanol suppressed the NO concentration by 87 % when compared to the control. The extract reconstituted with water suppressed the NO concentration by 49 % when compared to the control.

3.2 TNF-$\alpha$ and interleukin 6 (IL 6) are cytokins that are stimulators for an inflammatory response just like LPS. Determination of production of pro-inflammatory cytokines using ELISA Assay: The cell culture medium supernatants were subjected to test for the concentration of cytokines, TNF-$\alpha$ and IL-6 with commercial mouse enzyme -linked immunosorbent assay (ELISA) kits according to manufacturer's instructions. The OD of microplate was read at 450nm.

3.2.1 Results obtained in experiment 3: Nitric Oxide (NO) Inhibitory assay and pro-inflammatory cytokines using ELISA assay

To determine the effect CSTE extracts on the levels of nitric oxide (NO), TNF-$\alpha$ and interleukin IL-6 secretion from RAW 264.7 cells by ELISA. Results showed that both extract reconstituted with ethanol and water did suppressed NO production in LPS-induced RAW 264.7 cells (Fig.3A and B) as compared to the control group without exposure to the extract (LPS).

In contrast, extract reconstituted with ethanol and water significantly supressed the NO production by 49% to 87% respectively (p<0.003), when compare to control with LPS (Fig. 3A and B). Raw 264.7 after stimulation with LPS increase production of NO, TNF-$\alpha$ and IL-6 as compare to normal control cells as shown in figure 3.

The extract reconstituted with water displayed the following inhibitory rates at 12.5, 25 and 200 $\mu$g/ml: 43%, 46% and 71% for TNF-$\alpha$, and 67%, 49% and 50% at 50 $\mu$g/ml concentration for IL-6 (Fig. 3C and D). However, extract reconstituted with ethanol slightly inhibited the secretion of TNF-$\alpha$ and IL-6, with Inhibitory rates at 6.25, 12.5 and 25 $\mu$g/ml of 48%, 43% and 54% for IL-6, no further reduction was detected when concentration was increased to 200 $\mu$g/ml.

Experiment 4: Determining the effect of extract (as described above) on collagen type 11 in porcine articular cartilage

4.1 Tissue acquisition for cell culture

Porcine stifle (knee joints) from three-month-old pigs were obtained from an abattoir and dissected under aseptic conditions to expose the femoral condyles, as described previously [20]. The superficial zone cartilage (100 $\mu$m) of the femoral condyles was harvested using a dermatome (Integra, Plainsboro, NJ, USA) and middle zone cartilage (1.25 mm slices) was removed from each plug using a custom cutting jig.

4.2 Monolayer culture

The superficial and middle zone was digested with 0.2% collagenase- P (Roche Pharmaceuticals, Nutley, NJ, USA) for three hours. Chondrocytes were plated as monolayers at a density of $1 \times 105$ cells/well in 12-well culture plates and incubated at 37°C in a humid atmosphere of 5% carbon dioxide and 95% air in DMEM/F-12 medium containing 1% fetal bovine serum. The next day the medium was changed to serum-free DMEM/F-12 medium with insulin transferrin selenium + Premix (BD Bioscience, Bedford, MA, USA). Cells were treated for four days with various concentrations of bark or root extract reconstituted in water and ethanol respectively and resveratrol (RSV) at 20$\mu$m was used as positive control. Cells and media were collected on day 4 for analysis

4.2 Enzyme-linked immunosorbent assay analysis of Collagen type II protein levels

An ELISA kit was used for quantitative determination of collagen type II(MD Biosciences, St. Paul, MN, USA) levels in the conditioned culture media. The assay is based on the competitive inhibition of primary antibody binding to collagen type II-coated plates.

4.3 Results:

Enzyme-linked immunosorbent assay analysis of Collagen type II protein levels

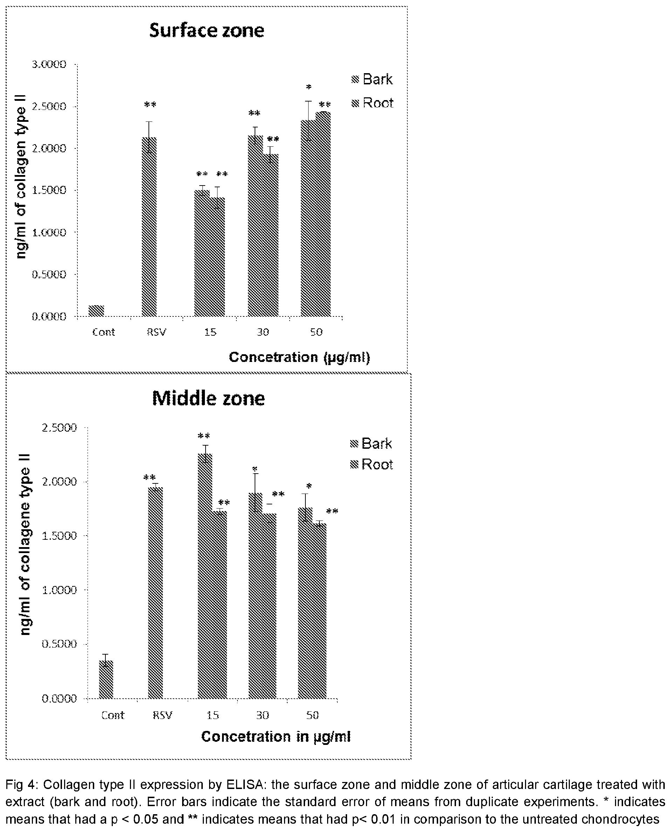

It was found that the expression levels of type II collagen gradually increased in a concentration-dependent manner when compared with those in the control chondrocytes without treatment. However, at a high concentration (50 $\mu$g/ml), the expression level of type II collagen was observed to be reduced (Fig. 5). When cells were treated with 15 $\mu$g/ml extracts, type II collagen expression levels were increased in both surface and middle zones more than positive control resveratrol (20 $\mu$M)(Fig. 4). These results could be attributed to collagen type II being localized more in the middle zone than in the surface zone.

## Discussion and conclusion

In the experiments, it was found that both extract reconstituted with water and ethanol respectively promote RAW 624.7 proliferation significantly. Anti-inflammatory control is one of the principles in the treatment of inflammation as it controls the swelling and relieves the pain from fracture sites and surrounding tissue which promote overall healing (Siu et al., 2015b; Zhang & An, 2007). During inflammation, macrophages and other immune cells activate overproduction of TNF and pro-inflammatory cytokines which are associated with development of inflammatory disorders (Rosas-Ballina et al., 2015; Zhang & An, 2007). Therefore, any plant-derived bioactive agent that can either prevent or neutralize excessive production of pro-inflammatory cytokine release is an important therapeutic strategy in inflammatory diseases.

The experiments demonstrate that the extract of *Capparis Sepiaria* and *Tabernaemontana Elegans* suppressed the production of pro-inflammatory cytokine nitric oxide (NO), IL-6 and TNF-$\alpha$ in macrophage RAW364.7 cells induce with LPS. The extract induces proliferation rate in normal RAW264.7 macrophage cells. TNF-$\alpha$ is one of the most important mediator for producing an inflammatory response that can induce acute inflammatory disease and cause tissue destruction (Fan et al., 2015). Blockage of LPS reduces production of pro-inflammatory cytokines. The inhibitory effect is largely related to the ability of the molecules involved in the medicinal plants to down-regulate the inflammatory mediators such as NO, IL-6 and TNF-$\alpha$.

The experiments indicated that the extract significantly inhibits the secretion of NO, TNF-$\alpha$ and IL-6 in our in vitro study. These findings suggest that the extract may be a useful therapeutic candidate for the prevention or treatment of inflammatory disease. The extract stimulated expression of collagen type II in both the surface and middle zone. The present study reveals the effects of the extract on tissue regeneration of articular cartilage.

It is to be appreciated, that the invention is not limited to any specific example or experiment hereinbefore disclosed.

## References

BARTOSH, T. J. & YLOSTALO, J. H. 2014. Macrophage Inflammatory Assay.4(14).

BREDBENNER, T. L., MASON, R. L., HAVILL, L. M., ORWOLL, E. S. & NICOLELLA, D. P. 2015. Fracture Risk Predictions Based on Statistical Shape and Density Modeling of the Proximal Femur. Journal of Bone and Mineral Research, 30(1):197-197.

DZOYEM, J. P., ARO, A. O., MCGAW, L. J. & ELOFF, J. N. 2016. Antimycobacterial activity against different pathogens and selectivity index of fourteen medicinal plants used in southern Africa to treat tuberculosis and respiratory ailments. South African Journal of Botany, 1021//:70-74.

FAN, X., ZHANG, Y., DONG, H., WANG, B., JI, H. & LIU, X. 2015. Trilobatin attenuates the LPS-mediated inflammatory response by suppressing the NF-$\kappa$B signaling pathway. Food Chemistry, 1661/1/:609-615.

FRANCESCA, N., BARBERA, M., MARTORANA, A., SAIANO, F., GAGLIO, R., APONTE, M., et al. 2016. Optimised method for the analysis of phenolic compounds from caper (Capparis spinosa L.) berries and monitoring of their changes during fermentation. Food Chemistry, 1964/1/:1172-1179.

IRANSHAHI, M., ASKARI, M., SAHEBKAR, A. & ADJIPAVLOU-LITINA , D. 2015. Evaluation of antioxidant, anti-inflammatory and lipoxygenase inhibitory activities of the prenylated coumarin umbelliprenin. 20152015-12-13:5.

JIN, S. E., KIM, O. S., YOO, S.-R., SEO, C.-S., KIM, Y., SHIN, H.-K., et al. 2016. Anti-inflammatory effect and action mechanisms of traditional herbal formula Gamisoyo-san in RAW 264.7 macrophages. BMC Complementary and Alternative Medicine, 16(1):1-11.

KALPANA, B. & PRAKASH, M. 2015. Antibacterial activity of Capparis sepiaria L.(Capparidaceae) leaves and fruits. Int. J. Curr. Microbiol. Appl. Sci, 4(1): 1007-1012.

KARKI, R., PARK, C.-H. & KIM, D.-W. 2013. Extract of buckwheat sprouts scavenges oxidation and inhibits pro-inflammatory mediators in lipopolysaccharide-stimulated macrophages (RAW264.7). Journal of Integrative Medicine, 11(4), 7//:246-252.

KENNEDY, D. O. & WIGHTMAN, E. L. 2011. Herbal extracts and phytochemicals: plant secondary metabolites and the enhancement of human brain function. Advances in Nutrition: An International Review Journal, 2(1):32-50.

LEE, H. & LEE, D. G. 2015. Mode of action of bioactive phytochemicals, plant secondary metabolites, possessing antimicrobial properties.

LOW, M., KHOO, C. S., MÜNCH, G., GOVINDARAGHAVAN, S. & SUCHER, N. J. 2015. An in vitro study of anti-inflammatory activity of standardised Andrographis paniculata extracts and pure andrographolide. BMC Complementary and Alternative Medicine, 15(1):1.

MANSOOR, T. A., RAMALHO, R. M., MULHOVO, S., RODRIGUES, C. M. P. & FERREIRA, M. J. U. 2009. Induction of apoptosis in HuH-7 cancer cells by monoterpene and β-carboline indole alkaloids isolated from the leaves of Tabernaemontana elegans. Bioorganic & Medicinal Chemistry Letters, 19(15), 8/1/:4255-4258.

MCCARBERG, B. & GIBOFSKY, A. 2012. Need to Develop New Nonsteroidal Anti-Inflammatory Drug Formulations. Clinical Therapeutics, 34(9), 9//:1954-1963.

MURRAY, P. J. & WYNN, T. A. 2011. Protective and pathogenic functions of macrophage subsets. Nature reviews. Immunology, 11(11), 10/14:723-737.

ROSAS-BALLINA, M., VALDÉS-FERRER, S. I., DANCHO, M. E., OCHANI, M., KATZ, D., CHENG, K. F., et al. 2015. Xanomeline suppresses excessive pro-inflammatory cytokine responses through neural signal-mediated pathways and improves survival in lethal inflammation. Brain, Behavior, and Immunity, 442//:19-27.

SIU, W.-S., KO, C.-H., LAM, K.-W., WAT, E., SHUM, W.-T., LAU, C. B.-S., et al. 2015a. Evaluation of a Topical Herbal Agent for the Promotion of Bone Healing. Evidence-Based Complementary and Alternative Medicine, 2015:10.

SIU, W.-S., KO, C.-H., LAM, K.-W., WAT, E., SHUM, W.-T., LAU, C. B.-S., et al. 2015b. Evaluation of a Topical Herbal Agent for the Promotion of Bone Healing. Evidence-Based Complementary and Alternative Medicine, 2015.

WADOOD, A., GHUFRAN, M., JAMAL, S., NAEEM, M., KHAN, A. & GHAFFAR, R. 2013. Phytochemical analysis of medicinal plants occurring in local area of Mardan. Biochem Anal Biochem, 2(4):1-4.

ZHANG, J.-M. & AN, J. 2007. Cytokines, Inflammation and Pain. International anesthesiology clinics, 45(2), Spring:27-37.

ZHONG, Y., CHIOU, Y.-S., PAN, M.-H. & SHAHIDI, F. 2012. Anti-inflammatory activity of lipophilic epigallocatechin gallate (EGCG) derivatives in LPS-stimulated murine macrophages. Food Chemistry, 134(2), 9/15/:742-748.

## Claims

1. A medicinal composition having anti-inflammatory properties including an extract derived from the root and bark of *Capparis Sepiaria* and *Tabernaemontana Elegans.*

2. A medicinal composition as claimed in claim 1, wherein the composition is in the form of a topical application and additionally includes:

   - a carbomer;
   - water;
   - a preservative;
   - Ethanol; and
   - Propylene glycol.

3. A medicinal composition as claimed in claim 2, wherein the preservative is DMDM Hydantoin.

4. A medicinal composition as claimed in claim 2 or claim 3, wherein the composition includes the following constituents, in mass percent:

   - Active Extract: 0.252 %;
   - Carbomer: 0.855%;
   - Water: 69.503%;
   - DMDM Hydantoin: 0.26 7%;
   - Ethanol 95 M5: 21.386 %; and
   - Propylene glycol: 7.485%.

5. A medicinal composition as claimed in any one of claims 2 to 4, wherein the constituents are combined and then mixed with a carrier selected from the group including: a gel, a spray, a paste, and a cream as are commonly used in cosmetic applications.

6. A medicinal composition as claimed in claim 5, wherein the carrier is a gel and the composition includes, in mass percent, 99.14457 % of the constituents as claimed in claim 4 and 0.85542 % gel.

7. A medicinal composition as claimed in any one of claims 1 to 6, for use in relieving/ assisting one or more of: muscle aches, joint aches, localised pain management, and wound healing in mammals.

8. A process for preparing an active extract for inclusion in a medicinal composition as claimed in any one of claims 1 to 7, including at least the steps of:

   - washing root and bark of *Capparis Sepiaria* and *Tabernaemontana Elegans* with water;
   - allowing the washed root and bark to dry at room temperature for about two weeks;
   - grinding and then mixing the dried root and bark to obtain a powder;
   - mixing the powder with distilled water and placing the resulting solution in a shaker for about 24 hours;
   - filtering the shaken solution using filter paper to obtain a filtrate;
- freezing the filtrate at a temperature of - 70 °C; and
- placing the frozen filtrate in a lyophiliser for four days so as to obtain the active extract.

9. A process as claimed in claim 8, wherein *Capparis Sepiaria* and *Tabernaemontana Elegans* are present in substantially equal amounts of which 50 % by weight is root and 50 % by weight is bark.

**Patentansprüche**

1. Medizinische Zusammensetzung, die entzündungshemmende Eigenschaften hat, beinhaltend ein Extrakt, das von der Wurzel und Rinde von *Capparis Sepiaria* und *Tabernaemontana Elegans* abgeleitet ist.

2. Medizinische Zusammensetzung nach Anspruch 1, wobei die Zusammensetzung in der Form einer topikalen Anwendung ist und zusätzlich Folgendes beinhaltet:

   - ein Carbomer;
   - Wasser;
   - ein Konservierungsmittel;
   - Ethanol; und
   - Propylenglykol.

3. Medizinische Zusammensetzung nach Anspruch 2, wobei das Konservierungsmittel DMDM-Hydantoin ist.

4. Medizinische Zusammensetzung nach Anspruch 2 oder Anspruch 3, wobei die Zusammensetzung die folgenden Bestandteile, in Massenprozent, beinhaltet:

   - Aktives Extrakt: 0,252%;
   - Carbomer: 0,855%;
   - Wasser: 69,503%;
   - DMD-Hydantoin: 0,267%;
   - Ethanol 95 M5: 21,386%; und
   - Propylenglykol: 7,485%.

5. Medizinische Zusammensetzung nach einem der Ansprüche 2 bis 4, wobei die Bestandteile kombiniert werden und dann mit einem Trägerstoff gemischt werden, der aus der Gruppe ausgewählt ist, beinhaltend: ein Gel, ein Spray, eine Paste und eine Creme, wie sie für gewöhnlich in kosmetischen Anwendungen verwendet werden.

6. Medizinische Zusammensetzung nach Anspruch 5, wobei der Trägerstoff ein Gel ist und die Zusammensetzung, in Massenprozent, 99,14457% der Bestandteile nach Anspruch 4 und 0,85542% Gel beinhaltet.

7. Medizinische Zusammensetzung nach einem der Ansprüche 1 bis 6, zur Verwendung beim Lindern/Unterstützen eines oder mehrerer von: Muskelschmerzen, Gelenkschmerzen, lokaler Schmerzbekämpfung und Wundheilung bei Säugern.

8. Prozess zum Zubereiten eines aktiven Extrakts zum Einschluss in eine medizinische Zusammensetzung nach einem der Ansprüche 1 bis 7, beinhaltend mindestens die folgenden Schritte:

   - Waschen von Wurzel und Rinde von *Capparis Sepiaria und Tabernaemontana Elegans* mit Wasser;
   - Trocknenlassen der gewaschenen Wurzel und Rinde, bei Raumtemperatur für etwa zwei Wochen;
   - Mahlen und dann Mischen der getrockneten Wurzel und Rinde, um ein Pulver zu erhalten;
   - Mischen des Pulvers mit destilliertem Wasser und Platzieren der resultierenden Lösung in einem Rüttler für etwa 24 Stunden;
   - Filtern der gerüttelten Lösung unter Verwendung von Filterpapier, um ein Filtrat zu erhalten;
   - Frieren des Filtrats bei einer Temperatur von -70°C; und
   - Platzieren des gefrorenen Filtrats in einem Gefriertrockner für vier Tage, um das aktive Extrakt zu erhalten.

9. Prozess nach Anspruch 8, wobei *Capparis Sepiaria* und *Tabernaemontana Elegans* in im Wesentlichen gleichen Mengen vorhanden sind, wobei 50 Gew.-% Wurzel und 50 Gew.-% Rinde sind.

**Revendications**

1. Composition médicinale possédant des propriétés anti-inflammatoires incluant un extrait dérivé de la racine et de l'écorce de *Capparis Sepiaria* et de *Tabernaemontana Elegans.*

2. Composition médicinale selon la revendication 1, dans laquelle la composition se présente sous la forme d'une application topique et inclut en outre :

   - un carbomère ;
   - de l'eau ;
   - un conservateur ;
   - de l'éthanol ; et
   - du propylène glycol.

3. Composition médicinale selon la revendication 2, dans laquelle le conservateur est de l'hydantoïne DMDM.

4. Composition médicinale selon la revendication 2 ou la revendication 3, dans laquelle la composition inclut les constituants suivants, exprimés en pourcentage en masse :

   - Extrait actif : 0,252 % ;
   - Carbomère : 0,855 % ;
   - Eau : 69,503 % ;
   - Hydantoïne DMDM : 0,267 % ;
   - Éthanol 95 M5 : 21,386 % ; et
   - Propylène glycol : 7,485 %.

5. Composition médicinale selon l'une quelconque des revendications 2 à 4, dans laquelle les constituants sont combinés et ensuite mélangés avec un support sélectionné dans le groupe comprenant : un gel, un spray, une pâte et une crème comme on en utilise habituellement dans les applications cosmétiques.

6. Composition médicinale selon la revendication 5, dans laquelle le support est un gel et la composition inclut, en pourcentage en masse, 99,14457 % des constituants selon la revendication 4 et 0,85542 % de gel.

7. Composition médicinale selon l'une quelconque des revendications 1 à 6, destiné à servir pour soulager / favoriser un ou plusieurs parmi : des douleurs musculaires, des douleurs articulaires, la prise en charge de douleurs locales et la cicatrisation chez les mammifères.

8. Procédé de préparation d'un extrait actif destiné à être incorporé dans une composition médicinale selon l'une quelconque des revendications 1 à 7, incluant au moins les étapes consistant à :

   - laver la racine et l'écorce de Capparis *Sepiaria et de Tabernaemontana Elegans* à l'eau ;
   - laisser sécher la racine et l'écorce lavées à température ambiante pendant environ deux semaines ;
   - broyer et ensuite mélanger la racine et l'écorce séchées pour obtenir une poudre ;
   - mélanger la poudre avec de l'eau distillée et placer la solution résultante dans un agitateur pendant environ 24 heures ;
   - filtrer la solution agitée au moyen d'un papier filtre pour obtenir un filtrat ;
   - congeler le filtrat à une température de -70 °C ; et
   - placer le filtrat congelé dans un lyophilisateur pendant quatre jours de façon à obtenir l'extrait actif.

9. Procédé selon la revendication 8, dans lequel Capparis *Sepiaria* et *Tabernaemontana Elegans* sont présents dans des quantités sensiblement égales dont 50% en poids sont de la racine et 50% en poids sont de l'écorce.

**Figure 1**: Dose–response cell viability detected by MTT assay after stimulated with extract reconstituted with water and ethanol respectively at different concentrations from 24, 48, and 72 hours. Bars represent the corresponding standard deviations (n = 3).

**Figure 2**: RAW264.7 seeded in the E-Plate 16 for 24 hours were treated with CSTE extracts at different concentration. Cell cultures not treated with compound were used as control. Cell Index values were monitored every 30 minutes for 24, 48 and 72 hrs. Slope curve indicating the viability of the cells. Data are expressed as mean and standard deviation (error bar).

**Figure 3**: Effect of extract reconstituted with water and ethanol on the secretion of NO, TNF-α and IL-6 in RAW 264.7 macrophages. The cells were culture with different concentrations of extract reconstituted with water and ethanol in the presence of LPS. Data are expressed as mean and standard deviation (error bar). ** p< 0.005, and *** p < 0.01 versus LPS.

Fig 4: Collagen type II expression by ELISA: the surface zone and middle zone of articular cartilage treated with extract (bark and root). Error bars indicate the standard error of means from duplicate experiments. * indicates means that had a p < 0.05 and ** indicates means that had p< 0.01 in comparison to the untreated chondrocytes

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BARTOSH, T. J. ; YLOSTALO, J. H.** *Macrophage Inflammatory Assay,* 2014, vol. 4 (14
- **BREDBENNER, T. L. ; MASON, R. L. ; HAVILL, L. M. ; ORWOLL, E. S. ; NICOLELLA, D. P.** Fracture Risk Predictions Based on Statistical Shape and Density Modeling of the Proximal Femur. *Journal of Bone and Mineral Research,* 2015, vol. 30 (1), 197-197
- **DZOYEM, J. P. ; ARO, A. O. ; MCGAW, L. J. ; ELOFF, J. N.** Antimycobacterial activity against different pathogens and selectivity index of fourteen medicinal plants used in southern Africa to treat tuberculosis and respiratory ailments. *South African Journal of Botany,* 2016, vol. 1021, 70-74
- **FAN, X. ; ZHANG, Y. ; DONG, H. ; WANG, B. ; JI, H. ; LIU, X.** Trilobatin attenuates the LPS-mediated inflammatory response by suppressing the NF-κB signaling pathway. *Food Chemistry,* 2015, vol. 1661 (1), 609-615
- **FRANCESCA, N. ; BARBERA, M. ; MARTORANA, A. ; SAIANO, F. ; GAGLIO, R. ; APONTE, M. et al.** Optimised method for the analysis of phenolic compounds from caper (Capparis spinosa L.) berries and monitoring of their changes during fermentation. *Food Chemistry,* 2016, vol. 1964 (1), 1172-1179
- **IRANSHAHI, M. ; ASKARI, M. ; SAHEBKAR, A. ; ADJIPAVLOU-LITINA , D.** *Evaluation of antioxidant, anti-inflammatory and lipoxygenase inhibitory activities of the prenylated coumarin umbelliprenin,* 2015, vol. 12 (13), 5
- **JIN, S. E. ; KIM, O. S. ; YOO, S.-R. ; SEO, C.-S. ; KIM, Y. ; SHIN, H.-K. et al.** Anti-inflammatory effect and action mechanisms of traditional herbal formula Gamisoyo-san in RAW 264.7 macrophages. *BMC Complementary and Alternative Medicine,* 2016, vol. 16 (1), 1-11
- **KALPANA, B. ; PRAKASH, M.** Antibacterial activity of Capparis sepiaria L.(Capparidaceae) leaves and fruits. *Int. J. Curr. Microbiol. Appl. Sci,* 2015, vol. 4 (1), 1007-1012
- **KARKI, R. ; PARK, C.-H. ; KIM, D.-W.** Extract of buckwheat sprouts scavenges oxidation and inhibits pro-inflammatory mediators in lipopolysaccharide-stimulated macrophages (RAW264.7). *Journal of Integrative Medicine,* 2013, vol. 11 (4), 246-252

- **KENNEDY, D. O. ; WIGHTMAN, E. L.** Herbal extracts and phytochemicals: plant secondary metabolites and the enhancement of human brain function. *Advances in Nutrition: An International Review Journal,* 2011, vol. 2 (1), 32-50
- **LEE, H. ; LEE, D. G.** *Mode of action of bioactive phytochemicals, plant secondary metabolites, possessing antimicrobial properties,* 2015
- **LOW, M. ; KHOO, C. S. ; MÜNCH, G. ; GOVINDARAGHAVAN, S. ; SUCHER, N. J.** An in vitro study of anti-inflammatory activity of standardised Andrographis paniculata extracts and pure andrographolide. *BMC Complementary and Alternative Medicine,* 2015, vol. 15 (1), 1
- **MANSOOR, T. A. ; RAMALHO, R. M. ; MULHOVO, S. ; RODRIGUES, C. M. P. ; FERREIRA, M. J. U.** Induction of apoptosis in HuH-7 cancer cells by monoterpene and β-carboline indole alkaloids isolated from the leaves of Tabernaemontana elegans. *Bioorganic & Medicinal Chemistry Letters,* 2009, vol. 19 (15), 4255-4258
- **MCCARBERG, B. ; GIBOFSKY, A.** Need to Develop New Nonsteroidal Anti-Inflammatory Drug Formulations. *Clinical Therapeutics,* 2012, vol. 34 (9), 1954-1963
- **MURRAY, P. J. ; WYNN, T. A.** Protective and pathogenic functions of macrophage subsets. *Nature reviews. Immunology,* 2011, vol. 11 (11), 723-737
- **ROSAS-BALLINA, M. ; VALDÉS-FERRER, S. I. ; DANCHO, M. E. ; OCHANI, M. ; KATZ, D. ; CHENG, K. F. et al.** Xanomeline suppresses excessive pro-inflammatory cytokine responses through neural signal-mediated pathways and improves survival in lethal inflammation. *Brain, Behavior, and Immunity,* 2015, vol. 442, 19-27
- **SIU, W.-S. ; KO, C.-H. ; LAM, K.-W. ; WAT, E. ; SHUM, W.-T. ; LAU, C. B.-S. et al.** Evaluation of a Topical Herbal Agent for the Promotion of Bone Healing. *Evidence-Based Complementary and Alternative Medicine,* 2015, vol. 10
- **SIU, W.-S. ; KO, C.-H. ; LAM, K.-W. ; WAT, E. ; SHUM, W.-T. ; LAU, C. B.-S. et al.** Evaluation of a Topical Herbal Agent for the Promotion of Bone Healing. *Evidence-Based Complementary and Alternative Medicine,* 2015

- **WADOOD, A. ; GHUFRAN, M. ; JAMAL, S. ; NAEEM, M. ; KHAN, A. ; GHAFFAR, R.** Phytochemical analysis of medicinal plants occurring in local area of Mardan. *Biochem Anal Biochem,* 2013, vol. 2 (4), 1-4
- Cytokines, Inflammation and Pain. **ZHANG, J.-M. ; AN, J.** International anesthesiology clinics. Spring, 2007, vol. 45, 27-37
- **ZHONG, Y. ; CHIOU, Y.-S. ; PAN, M.-H. ; SHAHIDI, F.** Anti-inflammatory activity of lipophilic epigallocatechin gallate (EGCG) derivatives in LPS-stimulated murine macrophages. *Food Chemistry,* 2012, vol. 134 (2), 742-748